# EUROPÄISCHE PATENTANMELDUNG

(11) **EP 3 485 749 A1**
(43) Veröffentlichungstag der Anmeldung: **22.05.2019**
(21) Anmeldenummer: 18206835.3
(22) Anmeldetag: 16.11.2018
(51) Int. Cl.: A41D 13/008, A41D 13/05, A41D 1/21, G21F 3/02

(54) **STRAHLENSCHUTZKLEIDUNG, INSBESONDERE BAUCHBINDE, FÜR DEN SCHUTZ VOR MOBILFUNKSTRAHLUNG**

(30) Priorität: 16.11.2017 DE 202017106976 U
(71) Anmelder: Jasseb, Ali, 64546 Mörfelden (DE)
(72) Erfinder: Jasseb, Ali, 64546 Mörfelden (DE)
(74) Vertreter: Metten, Karl-Heinz

(57) **Zusammenfassung**

Die vorliegende Erfindung betrifft eine Strahlenschutzbekleidung (1), insbesondere Bauchbinde, vorzugsweise für den Schutz vor Mobilfunkstrahlung, umfassend eine Außenlage (6) und eine Innenlage (7), wobei die Strahlenschutzbekleidung (1) zwischen der Außenlage (6) und der Innenlage (7) einen Innenraum (8) mit einer Eingriffsöffnung (9) und eine Abschirmlage (10), insbesondere mit einem Strahlenschutztextil (11), aufweist, wobei die Abschirmlage (10) entfernbar in dem Innenraum (8) angeordnet ist.

## Beschreibung

Die Erfindung betrifft eine Strahlenschutzbekleidung, insbesondere Bauchbinde, welche Nutzer vor Mobilfunkstrahlung schützt.

Aus dem Stand der Technik ist Kleidung für den Schutz vor Strahlung hinreichend bekannt. Strahlenschutzanzüge für radioaktive Strahlung werden beispielsweise bei Unfällen in Kernkraftwerken eingesetzt. Daneben besteht jedoch auch ein ziviler Bedarf an Schutzkleidung mit einem höheren Tragekomfort, da Mobilfunkstrahlung Körpergewebe erwärmen und Zellschäden auslösen kann. Es ist ferner bekannt, dass besonders Föten vor der Geburt empfindlicher auf Umwelteinflüsse reagieren als Erwachsene. Daher besteht hier ein besonderer Bedarf für den Schutz vor Mobilfunkstrahlung.

Die US 5,621,188 offenbart beispielsweise eine Kleidung, die vor elektromagnetischer Strahlung schützen soll, wobei eine Vielzahl sphärischer Metallobjekte in ein Gewebe eingearbeitet ist.

Die DE 200 17 110 U1 offenbart ein Abschirmhemd, wobei das Gewebe des Abschirmhemdes aus einem strahlungsdämpfenden Stoff besteht.

Die DE 201 21102 U1 offenbart Strümpfe mit leitfähigem Gewebe, um Schutz vor Elektrosmog zu bieten.

Die US 6,266,824 B1 offenbart eine Kopfbedeckung aus Kupfer oder Bronze zum Schutz vor elektromagnetischer Strahlung.

Die US 5,073,984 offenbart eine Weste, eine Kappe oder einen Mantel mit einem metallbeschichteten Garn zum Schutz vor elektromagnetischer Strahlung.

Die US 5,103,504 offenbart Kleidung, welche vor elektromagnetischer Strahlung schützen soll und Stahlfasern umfasst.

Herkömmliche Kleidung, die vor Strahlung schützen soll, ist oftmals unbequem, beispielsweise wenn Bleiplatten verwendet werden. Kleidung, die effektiv schützt und zudem bequem ist, gibt es kaum. Meist ist der Schutz ungenügend oder nur von kurzer Dauer, da zu viele Kompromisse gemacht werden. Auch ist die Reinigung oftmals erschwert.

Die Aufgabe der vorliegenden Erfindung war es daher, eine verbesserte Strahlenschutzbekleidung, insbesondere Bauchbinde, bereitzustellen, welche hygienisch und bequem ist und zudem einen verbesserten Strahlenschutz bietet.

Die Aufgabe wird gelöst durch eine Strahlenschutzbekleidung, insbesondere Bauchbinde, vorzugsweise für den Schutz vor Mobilfunkstrahlung, umfassend eine Außenlage und eine Innenlage, wobei die Strahlenschutzbekleidung zwischen der Außenlage und der Innenlage einen Innenraum mit einer Eingriffsöffnung und eine Abschirmlage, insbesondere eine Abschirmlage umfassend ein Strahlenschutztextil, umfasst, wobei die Abschirmlage entfernbar in dem Innenraum angeordnet ist. Vorzugsweise umfasst die Strahlenschutzbekleidung auch ein oberes Ende, ein unteres Ende, einen ersten Seitenrand und einen gegenüberliegenden zweiten Seitenrand.

Die erfindungsgemäße Strahlenschutzbekleidung, insbesondere Bauchbinde, hat den Vorteil, dass die Abschirmlage sich entfernen lässt. Es hat sich gezeigt, dass die Abschirmlage einen erheblichen Anteil ihrer ursprünglichen abschirmenden Wirkung verliert, wenn diese gewaschen wird. Die genaue Ursache hierfür ist nicht untersucht worden; allerdings könnte es damit zusammenhängen, dass beispielsweise Bestandteile, insbesondere Silber, der Abschirmlage ausgewaschen und/oder chemisch verändert, insbesondere oxidiert, werden. Mit der vorliegenden Erfindung ist es vorgesehen, dass die Abschirmlage mit der Haut nicht unmittelbar in Kontakt kommt und somit auch nicht gewaschen werden muss. Sie lässt sich aus dem Innenraum herauslösen, so dass der Innenraum samt Außenanlage und Innenlage mit aus dem Stand der Technik bekannten Verfahren gewaschen werden können.

Die Innenlage im Sinne der vorliegenden Erfindung, ist eine Lage, insbesondere Stofflage, welche bei der gattungsgemäßen Verwendung als Körperbekleidung dem menschlichen Körper zugewandt ist, insbesondere an der Haut anliegt. Die Außenanlage im Sinne der vorliegenden Erfindung ist dementsprechend eine gegenüberliegende Lage, welche nicht unmittelbar oder zumindest überwiegend nicht unmittelbar mit der Haut in Kontakt steht, insbesondere vom menschlichen Körper bei der gattungsgemäßen Nutzung abgewandt ist. Zwischen diesen besagten Lagen ist der Innenraum ausgebildet; dieser wird vorzugsweise auch durch Innen- und Außenlage begrenzt, wobei es natürlich auch denkbar ist, dass weitere Lagen innenseitig oder außenseitig an die Innen- bzw. Außenanlage angrenzen. Vorzugsweise ist es jedoch vorgesehen, dass die Innenlage, die Außenanlage und die dazwischen liegende Abschirmlage zumindest abschnittsweise, insbesondere überwiegend, ein genau dreilagiges System bilden, aus welchem die Abschirmlage entfernbar ist.

Eine Strahlenschutzbekleidung im Sinne der vorliegenden Erfindung kann ein Bekleidungsstück oder Teil eines Bekleidungsstücks sein, wobei dieses Strahlung, insbesondere Mobilfunkstrahlung, ganz oder teilweise abschirmt. Insbesondere bevorzugt ist es ein Kleidungsstück, das dazu ausgelegt und eingerichtet ist, den menschlichen Torso und/oder Oberkörper ganz oder teilweise zu umschließen. Vorzugsweise handelt es sich um ein Bekleidungsstück, beispielsweise eine Weste, eine Unterhose, ein T-Shirt, ein Hemd oder ein Unterhemd. Als ganz besonderes geeignet hat sich als Bekleidungsstück eine Bauchbinde erwiesen.

Eine Bauchbinde im Sinne der vorliegenden Erfindung ist ein Tuch, welches Schwangere um den Bauch fixiert tragen können und das den Bauch bedeckt. Üblicherweise und vorzugsweise bleiben der Brustkorb und die Beine dabei unbedeckt von der Bauchbinde. Eine Bauchbinde kann auch als Bauchtuch oder Bauchband bezeichnet werden. Die erfindungsgemäße Strahlenschutzbekleidung ist vorzugsweise eine solche Bauchbinde.

Das obere Ende im Sinne der vorliegenden Erfindung ist vorzugsweise jenes Ende, welches aus Richtung der Füße in Richtung des Kopfes des Trägers des Kleidungsstücks deutet, wenn das Kleidungsstück gattungsgemäß getragen wird. Bei einer Bauchbinde liegt das obere Ende oft im Brustbereich oder kurz unterhalb des Brustbereichs an.

Strahlung im Sinne der vorliegenden Erfindung ist in erster Line und vorzugsweise Mobilfunkstrahlung, insbesondere bei 900 MHz, 1800 MHz und/oder bei 2600 MHz.

In einer Ausgestaltung ist es vorgesehen, dass die Eingriffsöffnung reversibel verschließbar ist, beispielsweise durch einen Reißverschluss. Eine Verschließbarkeit der Eingriffsöffnung hat den Vorteil, dass die Abschirmungslage besser vor Umwelteinflüssen geschützt ist, was die Lebensdauer verlängert. Hierbei kann es vorgesehen sein, dass die Eingriffsöffnung mit einem Verschlussmittel verschließbar ist, wobei das Verschlussmittel ein Reißverschluss, ein Klettverschluss, ein Schnürverschluss, ein magnetischer Verschluss, ein Hakenverschluss oder ein Knopfverschluss ist oder umfasst. Als besonders geeignet hat sich ein Reißverschluss erwiesen.

Ferner ist es bevorzugt, wenn das Verschlussmittel durch einen Stoffschutzstreifen verdeckt werden kann. Dies vermindert die Verletzungsgefahr durch die Verschlussmittel. Auch wird die Gefahr einer Verunreinigung vermindert. Die Verschlussmittel umfassen meist mechanische Teile, die miteinander wechselwirken (vgl. z.B. Reißverschluss), wobei diese Teile auch Verunreinigungen aufnehmen können. Ein Stoffschutzstreifen hat sich hier als zweckdienlich erwiesen. Der Stoffschutzstreifen kann einstückig mit der Außen- oder Innenlage verbunden sein oder gesondert aufgebracht sein.

Vorzugsweise ist es vorgesehen, dass die Eingriffsöffnung eine Längserstreckung aufweist, welche mindestens 20 %, insbesondere mindestens 40 %, vorzugsweise mindestens 60 %, des mittleren Abstands zwischen dem oberen und unteren Ende entspricht. Vorzugsweise entspricht die Längserstreckung weniger als 100%, insbesondere weniger als 90%, des mittleren Abstands zwischen dem oberen und unteren Ende. Eine Eingriffsöffnung mit derartigen Maßen erlaubt eine besonders einfache Entfernung der Abschirmlage, wobei gleichzeitig die Stabilität der Eingriffsöffnung hoch ist.

In einer zweckmäßigen Ausgestaltung ist es vorgesehen, dass die Eingriffsöffnung einen Eingriffsöffnungsspalt, insbesondere mit der vorstehend beschriebenen Längserstreckung, umfasst oder darstellt, der sich zumindest abschnittsweise, vorzugsweise im Wesentlichen vollständig, parallel zum ersten und/oder zweiten Seitenrand erstreckt. Obgleich auch eine horizontale Erstreckung parallel zum unteren und/oder oberen Ende denkbar ist, hat sich eine Längserstreckung zumindest abschnittsweise, vorzugsweise im Wesentlichen vollständig, parallel zum ersten und/oder zweiten Seitenrand als besser erwiesen. Die Bauchbinde sollte vorzugsweise dehnbar sein, insbesondere wobei das Strahlenschutztextil in einer geeigneten Ausgestaltung entfaltet wird. Diese Entfaltung kann durch eine Längserstreckung parallel zum unteren und/oder oberen Ende eingeschränkt oder gar verhindert werden. Daher ist die vorstehend genannte Orientierung bevorzugt.

In einer weiteren Ausführungsform weist die erfindungsgemäße Strahlenschutzbekleidung am ersten Rand mindestens ein erstes Befestigungsmittel auf, welches ausgelegt und eingerichtet ist, mit mindestens einem zweiten Befestigungsmittel des zweiten Seitenrandes verbunden zu werden. Vorzugsweise werden die ersten und zweiten Seitenränder dabei so verbunden, dass die Stoffe der Innen- und Außenlage zumindest teilweise überlappen, da dies einen verbesserten Strahlenschutz im Verbindungsbereich bewirkt. Ferner ist es vorzugsweise vorgesehen, dass die erfindungsgemäße Strahlenschutzbekleidung an dem ersten Seitenrand mindestens ein erstes Befestigungsband mit einer Vielzahl von ersten Befestigungsmitteln aufweist, insbesondere mindestens drei ersten Befestigungsmitteln, vorzugsweise mindestens zehn ersten Befestigungsmitteln, und/oder das die Strahlenschutzbekleidung an dem zweiten Seitenrand mindestens ein zweites Befestigungsband mit einer Vielzahl von zweiten Befestigungsmitteln aufweist, insbesondere mindestens drei zweiten Befestigungsmitteln, vorzugsweise mindestens zehn zweiten Befestigungsmitteln. Durch die Vielzahl an Befestigungsmitteln wird sichergestellt, dass die Befestigung gleichmäßig und sicher ohne Druckpunkte auf dem Körper der Nutzerin erfolgt.

Ferner kann es vorgesehen sein, dass die erfindungsgemäße Strahlenschutzbekleidung ein drittes Befestigungsband mit einer Vielzahl von dritten Befestigungsmitteln, ein viertes Befestigungsband mit einer Vielzahl von vierten Befestigungsmitteln und/oder ein fünftes Befestigungsband mit einer Vielzahl von fünften Befestigungsmitteln aufweist, insbesondere dritte, vierte, fünfte Befestigungsbänder mit jeweils mindestens drei dritten, vierten bzw. fünften Befestigungsmitteln, vorzugsweise mindestens zehn dritten, vierten bzw. fünften Befestigungsmitteln. Vorzugsweise verlaufen das zweite, dritte, vierte und/oder fünfte Befestigungsband parallel und/oder beabstandet zueinander, insbesondere parallel und beabstandet. Durch den Einsatz mehrere Befestigungsbänder kann der Umfang der Strahlenschutzbekleidung an den Körperumfang angepasst werden, was insbesondere bei Schwangeren bedeutsam ist. Auch ist es bevorzugt, wenn das erste, zweite, dritte, vierte und/oder fünfte Befestigungsband beabstandet und/oder parallel zu ersten und/oder zweiten Seitenrand orientiert ist.

In einer Ausgestaltung kann auch die Abschirmlage mehrere Schichten umfassen. Dies verbessert den Strahlenschutz. Vorzugsweise umfasst die Abschirmlage aber nur eine Schicht. Auch ist es denkbar, dass weitere Abschirmlage an die vorstehend beschriebene Abschirmlage angrenzen, insbesondere mit dieser Verbunden sind. Vorzugsweise ist in einer Ausgestaltung nur genau eine Abschirmlage vorgesehen.

Alternativ oder zusätzlich kann es vorgesehen sein, dass mindestens ein zweites Befestigungsband, insbesondere das vorstehend beschriebene zweite Befestigungsband, zum Einsatz kommt, wobei das mindestens eine zweite Befestigungsband zweite Befestigungsmittel umfasst und insbesondere breiter als das erste Befestigungsband ist, wobei besagtes zweite Befestigungsband mehrere Positionen aufweist an welchen sich das erste Befestigungsband verbinden lässt. In einer Ausgestaltung kann dies erreicht werden, indem das erste Befestigungsband und das zweite Befestigungsband korrespondierende Bestandteile eines Klettverschlusssystems sind, insbesondere wobei das erste Befestigungsband Haken des Klettverschlusssystems aufweist und das, vorzugsweise breitere, zweite Befestigungsband Schlaufen des Klettverschlusssystems. Auch die umgekehrte Anordnung von Haken und Schlaufen ist denkbar. Das erste Befestigungsband kann nun an verschiedenen Stellen des, vorzugsweise breiteren zweiten, Befestigungsbands befestigt werden. Dies funktioniert nicht oder vergleichsweise schlecht, wenn die Befestigungsbänder dieselbe Breite aufweisen. Vorzugsweise ist das zweite Befestigungsband mindestens dreimal, insbesondere fünfmal, so breit wie das erste Befestigungsband, wobei die Befestigungsbänder vorzugsweise neben einer Breite auch eine Länge und Dicke, insbesondere Materialstärke, aufweisen.

Vorzugsweise sind die ersten und zweiten Befestigungsmittel dafür ausgelegt und eingerichtet, die Strahlenschutzbekleidung umlaufend an einem Körperteil des menschlichen Körpers zu befestigen, insbesondere an einem Bauch. In einer Ausgestaltung kann es dabei vorgesehen sein, dass das erste Befestigungsmitte an der Innenlage und das zweite sowie ggf. dritte, vierte und/oder fünfte Befestigungsmittel an der Außenlage angebracht sind. Alternativ kann es vorgesehen sein, dass das erste Befestigungsmitte an der Außenlage und das zweite sowie ggf. dritte, vierte oder fünfte Befestigungsmittel an Innenlage der angebracht sind. Hierbei ist es bevorzugt, wenn Innenlage und Außenlage sich überschneiden, wenn die korrespondierenden Befestigungsmittel miteinander verbunden werden.

Das Befestigungsband kann ein Band mit Druckknöpfen, Miederhaken und -ösen, Klettverschlusshaken und -schlingen und vieles mehr sein. Vorzugsweise umfassen die Befestigungsmittel, insbesondere die ersten, zweiten, dritten, vierten und/oder fünften Befestigungsmittel, Haken, insbesondere Metallhaken oder Klettverschlusshaken, Ösen, Knöpfe, insbesondere Druckknöpfe, und/oder Schnürungen. Auch ein magnetischer Verschluss ist grundsätzlich denkbar. Als besonders geeignet für einen optimalen Schutz vor Strahlung haben sich allerdings Miederhaken oder - ganz besonders geeignet - Klettverschlüsse erwiesen.

Vorzugweise ist es vorgesehen, dass die Strahlenschutzbekleidung am oberen und/oder unteren Ende und/oder beabstandet von diesem, insbesondere am oberen Ende und/oder beabstandet von diesem, insbesondere mit einem Abstand von weniger als 5 cm, vorzugsweise weniger als 3 cm, ein Gummiband aufweist. Als besonders geeignet hat sich ein Gummiband aus Silikon erwiesen. Auch kann es vorgesehen sein, dass das Gummiband eine Rille aufweist. Vorzugsweise ist das Gummiband außenseitig angebracht, so dass es beim ordnungsgemäßen Tragen auf der Haut aufliegt. Insbesondere ist es bevorzugt, wenn das Gummiband in Körperrichtung eine glatte, strukturlose Oberfläche aufweist. Das beschriebene Gummiband versiegelt den Bereich zwischen Haut und Körper am oberen und gegebenenfalls auch am unteren Ende. Es wirkt demnach im Wesentlichen wie eine Dichtung. Hierbei hat sich überraschenderweise gezeigt, dass auf diesem Weg der Schutz vor Mobilfunkstrahlung verbessert werden kann. Die Strahlung kann nun nicht mehr durch einen Spalt zwischen Haut und Außenlage eindringen. Für einen optimalen Strahlenschutz ist es zweckmäßig, wenn das Gummiband sich vom ersten Seitenrand oder aus Richtung des ersten Seitenrands bis zum zweiten Seitenrand oder in Richtung des zweiten Seitenrands erstreckt.

In einer weiteren Ausgestaltung ist es vorgesehen, dass die Abschirmlage, vorzugsweise randständig, mindestens ein erstes Abschirmlagefixierungsmittel aufweist, welches ausgelegt und eingerichtet ist, mit mindestens einem zweiten Abschirmlagefixierungsmittel des Innenraums und/oder der Außenlage und/oder der Innenlage verbunden zu werden. Grundsätzlich ist es denkbar, die Abschirmlage ohne ein gesondertes Fixierungsmittel einzubringen. Hierbei kann jedoch nur schlecht verhindert werden, dass die Abschirmlage sich im Laufe des Tages innerhalb des Innenraums unkontrolliert bewegt, beispielsweise schwerkraftgetrieben zusammenfällt. Zwar geschieht dies oftmals nicht sofort, da die Reibung der Abschirmlage mit den Seitenwänden des Innenraums, insbesondere der Innenlage und der Außenlage, dem entgegenwirkt. Allerdings ist es für einen optimalen und langfristigen Strahlenschutz besser, wenn die Abschirmlage, wie vorstehend beschrieben, mit Abschirmlagefixierungsmitteln fixiert wird.

Hierbei kann es vorgesehen sein, dass der Innenraum einen, insbesondere randständigen, Bereich mit Innenraumnähten umfasst, wobei die zweiten Abschirmlagefixierungsmittel mit besagtem Bereich verbunden sind, insbesondere mit den Innenraumnähten in Kontakt stehen, vorzugsweise wobei besagte Innenraumnähte die Innenlage und die Außenlage miteinander verbindet. Es hat sich gezeigt, dass derartig befestigte Abschirmlagefixierungsmittel besonders sicher halten, wobei die Innenraumnähte doppelt genutzt werden und zudem die Abschirmlagefixierungsmittel durch die miteinander verbundenen Bereiche von Innenlage und Außenlage besonders gut stabilisiert werden.

Vorzugsweise ist es vorgesehen, dass die Abschirmlage, insbesondere randständig, einen ersten elastischen Gewebestreifen umfasst, wobei das mindestens eine erste Abschirmlagefixierungsmittel an dem ersten elastischen Gewebestreifen befestigt ist und/oder dass der Innenraum einen zweiten elastischen Gewebestreifen umfasst, wobei das mindestens eine zweite Abschirmlagefixierungsmittel an dem zweiten elastischen Gewebestreifen befestigt ist. Dies verbessert den Strahlenschutz erheblich. Die Gewebestreifen kontrollieren die Dehnung, falls die Strahlenschutzbekleidung, insbesondere Bauchbinde samt Abschirmlage, gedehnt wird. Wichtig ist dabei, dass das Strahlenschutztextil nicht oder zumindest nicht unkontrolliert gedehnt wird und insbesondere auch nicht dehnbares Strahlenschutztextil eingesetzt werden kann. Eine Dehnung von Strahlenschutztextil ist mit einer erheblichen Vergrößerung der Strahlungsdurchlässigkeit verbunden, wobei die Strahlung oftmals kaum noch effektiv nach einer Dehnung zurückgehalten wird.

Auch kann es vorgesehen sein, dass mindestens fünf, vorzugsweise mindestens zehn, insbesondere mindestens zwanzig, erste und/oder zweite Abschirmlagefixierungsmittel vorgesehen sind, insbesondere wobei die Anzahl der ersten Abschirmlagefixierungsmittel der Anzahl der zweiten Abschirmlagefixierungsmittel entspricht. Vorzugsweise sind die Abschirmlagefixierungsmittel umlaufend an der Abschirmlage angeordnet, zumindest jedoch in Richtung des oberen Endes vorgesehen. Bei dieser Anordnung wird eine besonders gute Fixierung gewährleistet.

Vorzugsweise ist es vorgesehen, dass die ersten Abschirmlagefixierungsmittel, insbesondere mit regelmäßigen Abständen, beabstandet voneinander auf einem bzw. dem ersten elastischen Gewebestreifen angeordnet sind und/oder dass die zweiten Abschirmlagefixierungsmittel, insbesondere mit regelmäßigen Abständen, beabstandet voneinander auf einem bzw. dem zweiten elastischen Gewebestreifen angeordnet sind. Dies hat den Vorteil, dass die Position der Abschirmlagefixierungsmittel sich entsprechend verschiebt, wenn die beiden Gewebestreifen sich gleichzeitig dehnen. Die an sich selbst meist nicht dehnbaren Abschirmlagefixierungsmittel, beispielsweise Druckknöpfe, werden also gemeinsam verschoben, so dass die ersten und zweiten Abschirmlagefixierungsmittel bei einer Dehnung aneinander haften bleiben und die sichere Fixierung der Abschirmlage erhalten bleibt.

Vorzugsweise ist es vorgesehen, dass die ersten und zweiten Abschirmlagefixierungsmittel jeweils gegenüberliegende Bestandteile von Knöpfen, insbesondere Druckknöpfen, sind. Insbesondere bevorzugt sind die Abschirmlagefixierungsmittel ferner überwiegend oder vollständig aus Kunststoff, insbesondere Druckknöpfe aus Kunststoff, wobei der maximale Durchmesser der Druckknöpfe vorzugsweise weniger als 0,8 cm, insbesondere weniger als 0,6 cm, beträgt. Es hat sich gezeigt, dass solche Abschirmlagefixierungsmittel besonders sicher halten, leicht zu fixieren sind (dies ist wichtig, da eine Fixierung im Innenraum erfolgen soll) und nicht auftragen (dies ist wichtig, da sonst Druckstellen am menschlichen Körper entstehen können).

Vorzugsweise ist es auch vorgesehen, wenn die Abschirmlage sich durch ein Umstülpen durch die Eingriffsöffnung hindurch nach außen drehen lässt. Dies erleichtert eine Fixierung der Abschirmlagefixierungsmittel erheblich. Während des Tragens der erfindungsgemäßen Strahlenschutzbekleidung erfolgt eine solche Umstülpung natürlich nicht.

Ferner kann es zweckmäßig sein, wenn die Abschirmlage, insbesondere randständig, einen ersten elastischen Gewebestreifen, vorzugsweise den vorstehend beschriebenen ersten elastischen Gewebestreifen, umfasst, wobei der erste elastische Gewebestreifen das Strahlenschutztextil der Abschirmlage umschließt, wobei das Strahlenschutztextil im relaxierten und/oder entspannten Zustand des ersten elastischen Gewebestreifens gerafft ist und/oder zahlreiche Falten aufweist und erst durch Dehnung des ersten elastischen Gewebestreifens in einen gedehnten und/oder gespannten Zustand ohne besagte Raffung bzw. mit weniger Falten überführbar ist, insbesondere wobei das Strahlenschutztextil weniger stark dehnbar als der erste elastischen Gewebestreifen ist. Dies verbessert den Strahlenschutz überraschenderweise erheblich. Wenn der erste und zweite elastische Gewebestreifen sich dehnt, entfaltet sich das Strahlenschutztextil, dehnt sich jedoch nicht oder nur in sehr geringen Maße. Eine Dehnung des Strahlenschutztextil hätte einen erheblichen Verlust der Stärke des Strahlenschutzes zur Folge. Die Ursache hierfür ist nicht erforscht worden, allerdings wäre zu vermuten, dass die größeren Lücken des gedehnten Gewebes durchlässiger für Strahlung sind. Diese Dehnung wird vorliegend allerdings effektiv unterbunden, da genug Material an Strahlenschutztextil bereitgestellt wird, so dass eine Dehnung nicht erfolgt. Bisherige Lösungen haben oft den negativen Effekt einer Dehnung des Strahlenschutztextils nicht hinreichend berücksichtigt. Mit der vorliegenden Lösung wird der Strahlenschutz daher erheblich verbessert und gleichzeitig eine Dehnung der erfindungsgemäßen Strahlenschutzbekleidung und gleichzeitige Entfaltung interner Elemente, d.h. des Strahlenschutztextils der Strahlenschutzbekleidung, insgesamt ermöglicht. Besagte Strahlenschutzbekleidung ist somit insgesamt dehnbar/entfaltbar und weist gleichwohl einen verbesserten Strahlenschutz auf.

In einer Ausführungsform sind die Innenlage und die Außenlage am oberen Ende und/oder am unteren Ende und/oder am ersten Seitenrand und/oder am zweiten Seitenrand durch, insbesondere elastische, Innenraumnähte, insbesondere mit elastischen Nähgarnen, miteinander verbunden, insbesondere wobei die Innenraumnähte auch den zweiten elastischen Gewebestreifen fixieren. Elastische Nähgarne erleichtern die vorstehend beschriebene Dehnung der Strahlenschutzbekleidung (nicht jedoch des Strahlenschutztextils).

Insbesondere bevorzugt ist auch ein Strahlenschutztextil mit einem Metalldrahtgeflecht, vorzugsweise umfassend oder bestehend aus Nickel, Silber, Kupfer und/oder Stahl. In einer Ausgestaltung ist es vorgesehen, dass das Strahlenschutztextil ein Silbergewebe umfasst oder darstellt. Derartige Strahlenschutztextile haben sich als besonders geeignet erwiesen. Auch ist es bevorzugt, wenn das Strahlenschutztextil eine Struktur aufweist, beispielsweise eine Rautenstruktur, da dies die Fixierung erleichtert und das Strahlenschutztextil aufgrund der größeren Reibung besser im Innenraum hält. Die Auswahl an möglichen Strahlenschutztextil wird vorliegend auch dadurch beeinflusst, dass diese entfernbar in einem Innenraum angeordnet sind, mithin ein direkter Hautkontakt nicht vorgesehen ist.

Vorzugsweise ist das Strahlenschutztextil antistatisch, antiseptisch, faltbar und/oder knickbar. Derartige Strahlenschutztextile haben sich als besonders geeignet für Kleidung erwiesen.

Auch ist es bevorzugt, dass das Flächengewicht des Strahlenschutztextils 10 bis 100 g/m², insbesondere 12 bis 50 g/m², beträgt. Wenn die Kleidung vollständig aus Abschirmgewebe hergestellt ist, hat sich ein solches Flächengewicht als weniger geeignet erwiesen, als wenn das Strahlenschutztextil - wie vorliegend - nur eine Lage in einem mehrlagigen System darstellt.

Vorzugsweise ist es vorgesehen, dass das Strahlenschutztextil (im ungedehnten und/oder relaxierten Zustand) bei 900 MHz, 1800 MHz und/oder bei 2600 MHz einen Schirmdämpfungswert von mindestens 10 dB, insbesondere mindestens 40 dB, vorzugsweise mindestens 50 dB, aufweist. Vorzugsweise wird der Schirmdämpfungswert gemäß MIL-STD 285 bestimmt. Vorzugsweise weist die Strahlenschutzbekleidung im geschlossenen Zustand im Innenraum bei 900 MHz, 1800 MHz und/oder bei 2600 MHz einen Schirmdämpfungswert von mindestens 50 dB auf.

Vorzugsweise liegt das Verschlussmittel in der Außenlage vor. Auch ein Verschlussmittel in der Innenlage ist denkbar, hat sich jedoch als weniger geeignet erwiesen, da eher Druckstellen beim gattungsgemäßen Tragen am menschlichen Körper erzeugt werden.

Vorzugsweise ist es vorgesehen, dass die Strahlenschutzbekleidung, insbesondere der Innenraum und/oder die Abschirmlage und/oder das Abschirmtextil, ausgelegt und eingerichtet sind, um den Torso oder Oberkörper einen Nutzers eilweise oder vollständig umlaufend angeordnet zu werden, insbesondere wobei der Innenraum und/oder die Abschirmlage und/oder das Abschirmtextil am Bauch und Rücken des Nutzers anliegt, insbesondere wobei kein Spalt oder nur ein schmaler Spalt mit einer Breite von weniger als 5 cm, insbesondere weniger als 2 cm, am Rücken frei bleibt.

Vorzugsweise ist es vorgesehen, das die Außenlage und/oder die Innenlage teilweise, insbesondere überwiegend, aus einem waschbaren Textil gebildet ist bzw. besteht, vorzugsweise ein Textil umfassend teilweise oder überwiegend Baumwolle. Das Material der Außenlage und/oder der Innenlage ist für den Strahlenschutz an sich unerheblich, sofern es geeignet ist, die Abschirmlage richtig zu orientieren und zu stabilisieren. Maßgeblich für den Strahlenschutz ist die Abschirmlage. Textile umfassend Baumwolle, Leinen, Ramie, Schafwolle oder andere Tierwolle, Polyester, Polyacryl, Elasthan, Viskose, Nylon und/oder Modal haben sich als geeignet erwiesen.

Der vorliegenden Erfindung liegt die Erkenntnis zugrunde, dass Abschirmlagen durch Waschen an Wirkung verlieren. Der Verzicht auf eine Reinigung der Abschirmlagen würde im Regelfall hygienischen Mindestanforderungen nicht entsprechen. Es konnte nunmehr eine Strahlenschutzbekleidung entwickelt werden, welche flexible, dehnbar und bequem ist und gleichzeitig Mobilfunkstrahlung effektiver abschirmt. Durch die Separierung in verschiedene Teile wird auch die Waschbarkeit gewährleistet. Insgesamt wird eine verbesserte Strahlenschutzbekleidung, insbesondere Bauchbinde bereitgestellt, welche hygienisch und bequem ist und zudem einen verbesserten Strahlenschutz bietet. Überraschenderweise hat sich ferner gezeigt, dass es besser ist, wenn die Strahlenschutzbekleidung nicht gedehnt wird. Auch hierfür konnte eine effektive Lösung gefunden werden, wie dies vorstehend beschrieben wurde.

Weitere Merkmale und Vorteile der Erfindung ergeben sich aus der nachfolgenden Beschreibung, in der Ausführungsbeispiele der Erfindung anhand von schematischen Zeichnungen beispielhaft erläutert werden, ohne dadurch die Erfindung zu beschränken.

Dabei zeigt:
- Figur 1: eine schematische Frontalansicht einer Nutzerin mit einer erfindungsgemä-ßen Strahlenschutzbekleidung;
- Figur 2: eine schematische Frontalansicht der Strahlenschutzbekleidung aus der Richtung der Außenlage;
- Figur 3: eine schematische Frontalansicht der Strahlenschutzbekleidung aus der zu Figur 2 gegenüberliegenden Richtung, d.h. der Richtung der Innenlage;
- Figur 4: eine schematische Frontalansicht der Abschirmlage, wenn diese aus dem Innenraum entfernt wird;
- Figur 5: eine schematische Ansicht einer Seite des Innenraums, wobei dies der ge-genüberliegenden Seite der Außenlage entspricht (die gegenüberliegende Seite der Innenlage wird in den Figuren nicht wiedergegeben);
- Figur 6: eine schematische vereinfachte perspektive Ansicht, wobei die Eingriffsöff-nung in der Außenlage geöffnet ist und die Abschirmlage teilweise hausgezo-gen wurde;
- Figur 7: einen schematischen Querschnitt durch die Strahlenschutzbekleidung an einer Position "A" wie in Figur 2 dargestellt;
- Figur 8: eine schematische Frontalansicht einer weiteren Ausgestaltung der Strahlen-schutzbekleidung; und
- Figur 9: eine schematische Frontalansicht einer weiteren Ausgestaltung der Strahlen-schutzbekleidung.

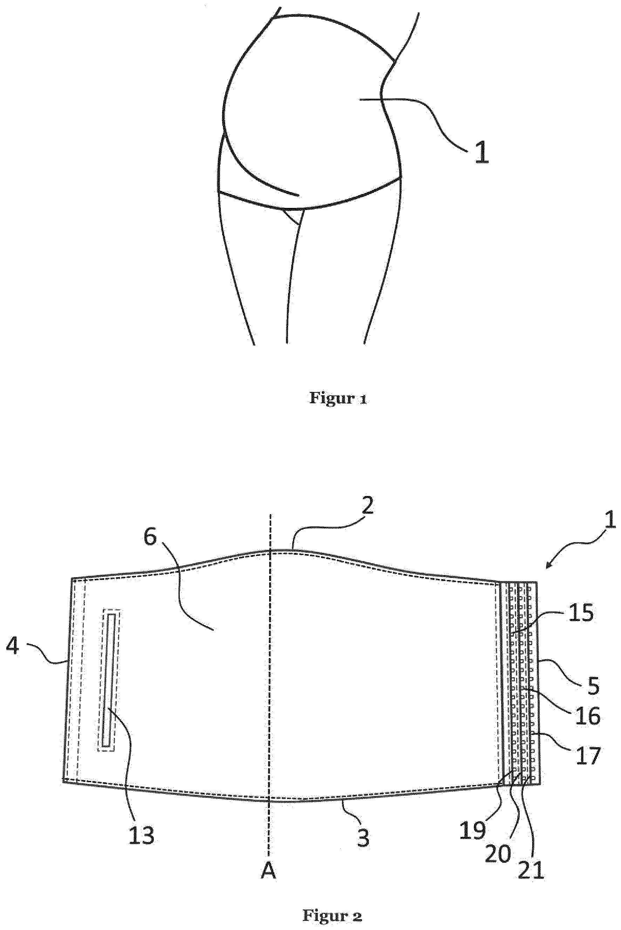

Figur 1 zeigt eine schematische Frontalansicht einer Nutzerin mit einer erfindungsgemäßen Strahlenschutzbekleidung 1. Die Strahlenschutzbekleidung ist in diesem Fall eine Bauchbinde.

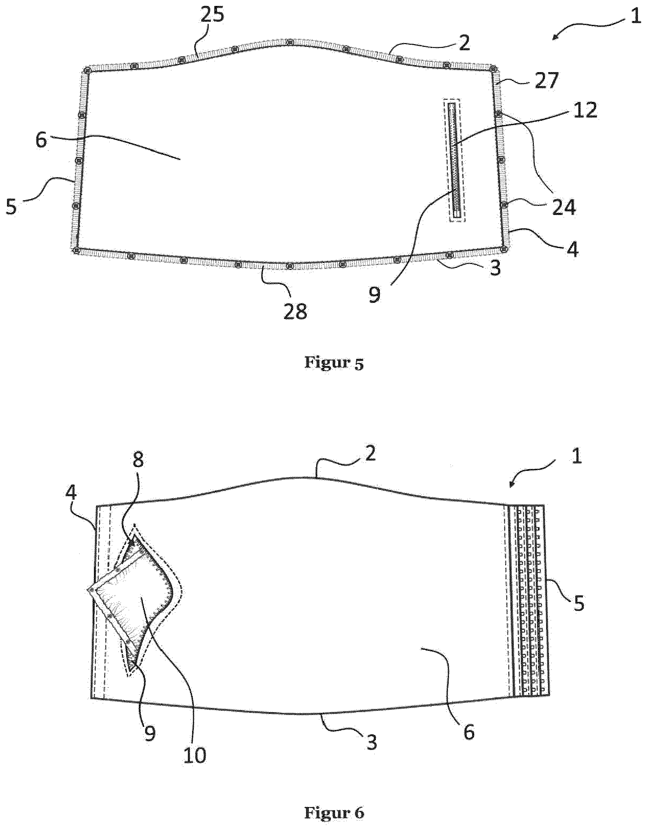

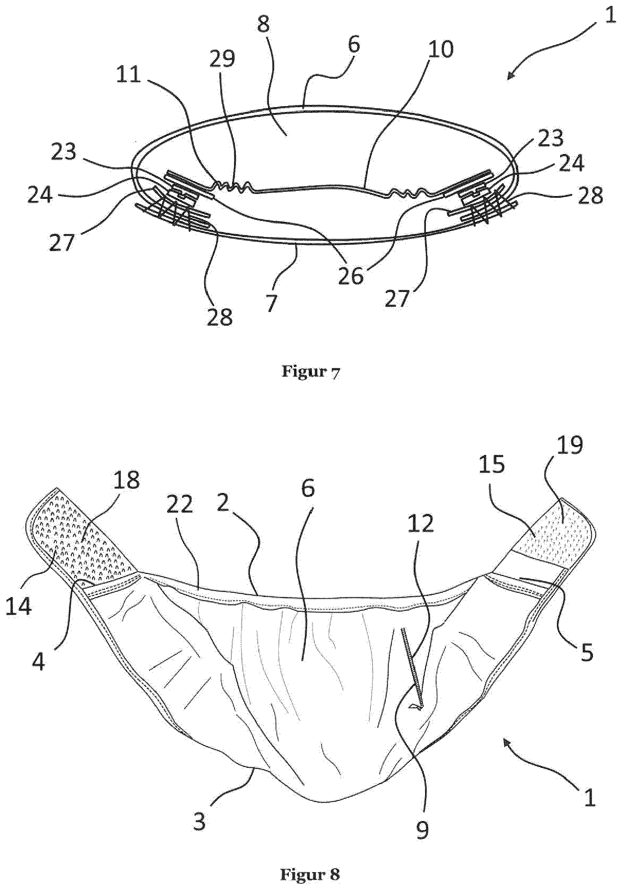

Figur 2 zeigt die Strahlenschutzbekleidung 1 in Form einer Bauchbinde für den Schutz vor Mobilfunkstrahlung, umfassend ein oberes Ende 2, ein unteres Ende 3, einen ersten Seitenrand 4 und einen gegenüberliegenden zweiten Seitenrand 5, eine Außenlage 6 und eine Innenlage (vgl. Figur 6, nicht gezeigt in Figur 1), wobei die Strahlenschutzbekleidung 1 zwischen Außenlage 6 und die Innenlage einen Innenraum (vgl. Figur 2 und 6, nicht gezeigt in Figur 1) mit einer Eingriffsöffnung umfasst. Das Verschlussmittel für die Eingriffsöffnung durch einen Stoffschutzstreifen 13 verdeckt. Die Strahlenschutzbekleidung 1 weist am ersten Seitenrand 4 erste Befestigungsmittel 14 auf, welche ausgelegt und eingerichtet sind, mit zweiten Befestigungsmitteln 15 des zweiten Seitenrandes 5 verbunden zu werden. Die Strahlenschutzbekleidung 1 weist ferner an dem ersten Seitenrand 4 ein erstes Befestigungsband 18 mit einer Vielzahl der besagten ersten Befestigungsmitteln 14 auf und an dem zweiten Seitenrand 5 ein zweites Befestigungsband 19 mit einer Vielzahl er besagten zweiten Befestigungsmittel 15. Auch zu sehen ist ein drittes Befestigungsband 20 mit einer Vielzahl von dritten Befestigungsmitteln 16 und ein viertes Befestigungsband 21 mit einer Vielzahl von vierten Befestigungsmitteln 17. Das zweite, dritte und vierte Befestigungsband 19, 20, 21 verlaufen parallel und beabstandet zueinander verlaufen und auch beabstandet und parallel zum zweiten Seitenrand 5. Der Querschnitt A ist in Figur 7 wiedergegeben.

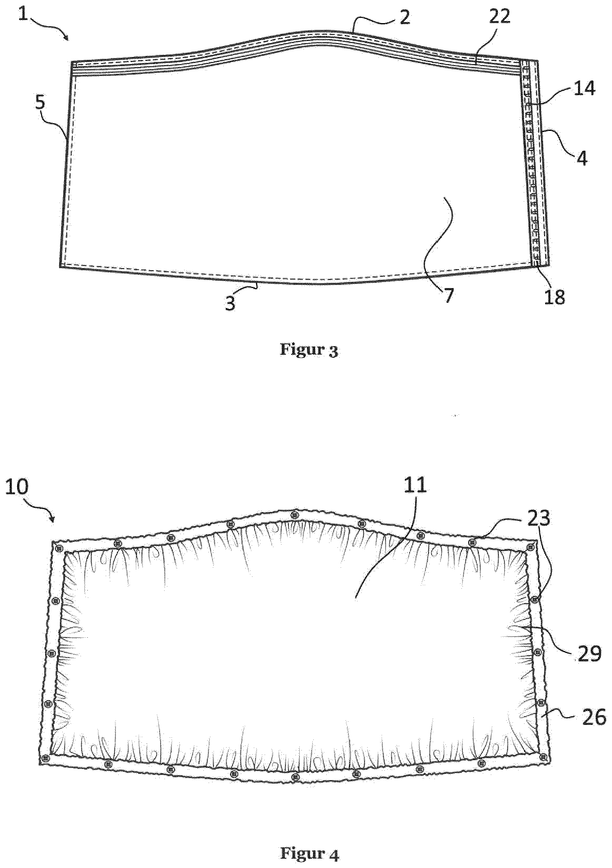

Figur 3 zeigt die Strahlenschutzbekleidung 1, insbesondere Bauchbinde, vorzugsweise für den Schutz vor Mobilfunkstrahlung, umfassend ein oberes Ende 2, ein unteres Ende 3, einen ersten Seitenrand 4 und einen gegenüberliegenden zweiten Seitenrand 5, eine Außenlage (vgl. Figur 1, nicht gezeigt in Figur 2) und eine Innenlage 7. Hierbei weist die Strahlenschutzbekleidung 1 an dem ersten Seitenrand 4 mindestens ein erstes Befestigungsband 18 mit einer Vielzahl von ersten Befestigungsmitteln 14 auf. Auch ist vorgesehen, dass die Strahlenschutzbekleidung 1 am oberen Ende 2 ein Gummiband 22 umfassend Silikon aufweist.

Figur 4 zeigt die Abschirmlage 10 mit einem Strahlenschutztextil 11, welche entfernbar in einem Innenraum angeordnet werden kann. Hierbei ist es vorgesehen, dass die Abschirmlage 10 randständig erste Abschirmlagefixierungsmittel 23 aufweist, welche ausgelegt und eingerichtet sind, mit zweiten Abschirmlagefixierungsmittel (vgl. Figur 5) verbunden zu werden. Die die Abschirmlage 10 weist randständig einen ersten elastischen Gewebestreifen 26 auf, wobei besagter erster elastischer Gewebestreifen 26 das Strahlenschutztextil 11 der Abschirmlage 10 umschließt, wobei das Strahlenschutztextil 11 im relaxierten und entspannten Zustand des ersten elastischen Gewebestreifens 26 gerafft ist und zahlreiche Falten 29 aufweist und erst durch Dehnung des ersten elastischen Gewebestreifens 26 in einem Zustand ohne besagte Raffung bzw. mit weniger Falten 29 überführbar ist, wobei das Strahlenschutztextil 11 weniger stark dehnbar als der erste elastischen Gewebestreifen 26 ist.

Figur 5 zeigt nochmals die Außenlage 6 aus Figur 2, wobei diese allerdings gewendet wurde, so dass die gegenüberliegende Seite zu sehen ist. Diese gegenüberliegende Seite begrenzt auch den Innenraum. Die Strahlenschutzbekleidung 1 umfasst ein oberes Ende 2, ein unteres Ende 3, einen ersten Seitenrand 4 und einen gegenüberliegenden zweiten Seitenrand 5 sowie besagte eine Außenlage 6. Die Außenlage 6 bzw. der durch die Außenlage 6 begrenzte Innenraum weist eine Eingriffsöffnung 9 auf, welche mit einem Verschlussmittel 12 verschließbar ist. Randständig sind die Innenraumnähte 28 zu sehen, wobei die zweiten Abschirmlagefixierungsmittel 24 in dem randständigen Bereich 25 mit besagten Innenraumnähten 28 vorgesehen sind. In diesem Bereich ist auch der zweite elastischen Gewebestreifen 27 umfasst, wobei das mindestens eine zweite Abschirmlagefixierungsmittel 24 an dem zweiten elastischen Gewebestreifen 27 befestigt ist. Der elastischen Gewebestreifen 27 wird durch die Innenraumnähten 28 fixiert.

Figur 6 zeigt nochmals die Außenlage 6 der Strahlenschutzbekleidung 1 der Figuren 2 und 5, wobei der Stoffschutzstreifen 13 nicht zu sehen ist und die Eingriffsöffnung 9 geöffnet wurde, um den Innenraum 8 zugänglich zu machen, so dass die Abschirmlage 10 durch die Eingriffsöffnung 9 hindurch entfernbar aus dem Innenraum 8 ist. Das obere Ende 2, das untere Ende 3, der ersten Seitenrand 4 und der gegenüberliegenden zweiten Seitenrand 5 sind wieder zu sehen.

Figur 7 zeigt einen schematischen Querschnitt durch die Strahlenschutzbekleidung 1 an einer Position "A" wie in Figur 2 gezeigt. Zu sehen ist die Strahlenschutzbekleidung 1 umfassend eine Außenlage 6 und eine Innenlage 7, wobei die Strahlenschutzbekleidung 1 zwi-schen Außenlage 6 und die Innenlage 7 einen Innenraum 8 mit einer Eingriffsöffnung 9 und eine Abschirmlage 10 mit einem Strahlenschutztextil 11 umfasst, wobei die Abschirm-lage 10 entfernbar in dem Innenraum 8 angeordnet ist. Die Abschirmlage 10 weist randständig mindestens ein erstes Abschirmlagefixierungsmittel 23 auf, welches ausgelegt und eingerichtet ist, mit mindestens einem zweiten Abschirmlagefixierungsmittel 24 des Innenraums 8 verbunden zu werden. Der Innenraum 8 umfasst einen randständigen Bereich mit Innenraumnähten 28, wobei die zweiten Abschirmlagefixierungsmittel 24 mit besagten Bereichen verbunden sind und mit den Innenraumnähten 28 in Kontakt stehen und auf diesen aufliegen, wobei besagte Innenraumnähte 28 die Innenlage 7 und die Außenlage 6 miteinander verbindet. Die Abschirmlage 10 umfasst randständig einen ersten elastischen Gewebestreifen 26, wobei das mindestens eine erste Abschirmlagefixierungsmittel 23 an dem ersten elastischen Gewebestreifen 26 befestigt ist und der Innenraum 8 umfasst einen zweiten elastischen Gewebestreifen 27, wobei das mindestens eine zweite Abschirmlagefixierungsmittel 24 an dem zweiten elastischen Gewebestreifen 27 befestigt ist. Die Abschirmlage 10 umfasst randständig den besagten ersten elastischen Gewebestreifen 26, wobei der erste elastische Gewebestreifen 26 das Strahlenschutztextil 11 der Abschirmlage 10 umschließt in der Querschnittsansicht nicht sichtbar (vgl. Figur 4), wobei das Strahlenschutztextil 11 im relaxierten und entspannten Zustand des ersten elastischen Gewebestreifens 26 gerafft ist und zahlreiche Falten 29 aufweist und erst durch Dehnung des ersten elastischen Gewebestreifens 26 in einem Zustand ohne besagte Raffung bzw. mit weniger Falten 29 überführbar ist, wobei das Strahlenschutztextil 11 weniger stark dehnbar als der erste elastischen Gewebestreifen 26 ist. Die Innenlage 7 und die Außenlage 6 sind am oberen Ende 2, am unteren Ende 3, am ersten Seitenrand 4 und/oder am zweiten Seitenrand 5 durch besagte elastische Innenraumnähte 28 miteinander verbunden, wobei die Innenraumnähte 28 auch den zweiten elastischen Gewebestreifen 27 fixieren.

Figur 8 zeigt eine Strahlenschutzbekleidung 1, insbesondere Bauchbinde, für den Schutz vor Mobilfunkstrahlung, vorzugsweise umfassend ein oberes Ende 2, ein unteres Ende 3, einen ersten Seitenrand 4 und einen gegenüberliegenden zweiten Seitenrand 5, ferner umfassend eine Außenlage 6 und eine Innenlage (gegenüber von der Außenlage, nicht gezeigt), wobei die Strahlenschutzbekleidung 1 zwischen der Außenlage 6 und der Innenlage 7 einen Innenraum mit einer Eingriffsöffnung 9 aufweist, wobei eine Abschirmlage entfernbar in dem Innenraum angeordnet ist (nicht gezeigt). Die Eingriffsöffnung 9 ist mit einem Verschlussmittel 12 verschließbar, wobei das Verschlussmittel 12 beispielsweise ein Reißverschluss sein kann. Die Strahlenschutzbekleidung 1 weist in der dargestellten Ausführungsform an dem ersten Seitenrand 4 anschließend mindestens ein erstes Befestigungselement, beispielsweise -band 18 mit einer Vielzahl von ersten Befestigungsmitteln 14 auf und an dem zweiten Seitenrand 5 mindestens ein zweites Befestigungselement, beispielsweise - band 19 mit einer Vielzahl von zweiten Befestigungsmitteln 15. Die ersten Befestigungsmittel 14 können, wie in Fig. 8 dargestellt, Ösen und die zweiten Befestigungsmittel 15 korrespondierende Klettverschlusshaken sein. Die in Figur 8 nicht gezeigte die Abschirmlage weist, vorzugsweise randständig, mindestens ein erstes Abschirmlagefixierungsmittel auf, welches ausgelegt und eingerichtet ist, mit mindestens einem zweiten Abschirmlagefixierungsmittel, das der ebenfalls nicht wiedergegebene Innenraum aufweist, verbunden zu werden. Hierbei kann es vorgesehen sein, dass die Abschirmlage, insbesondere randständig, einen ersten elastischen Gewebestreifen umfasst, wobei der erste elastische Gewebestreifen das Strahlenschutztextil der Abschirmlage umschließt insbesondere umläuft, wobei das Strahlenschutztextil in einem relaxierten und/oder entspannten Zustand des ersten elastischen Gewebestreifens gerafft ist und/oder mehrere Falten aufweist und erst durch Dehnung des ersten elastischen Gewebestreifens in einem Zustand ohne besagte Raffung bzw. mit weniger Falten überführbar ist, insbesondere wobei das Strahlenschutztextil weni-ger stark dehnbar als der erste elastischen Gewebestreifen ist (nicht gezeigt). Die Abschirmlage weist vorzugsweise randständig mindestens ein erstes Abschirmlagefixierungsmittel auf, welches ausgelegt und eingerichtet ist, mit mindestens einem zweiten Abschirmlagefixierungsmittel des Innenraums verbunden zu werden (nicht gezeigt).

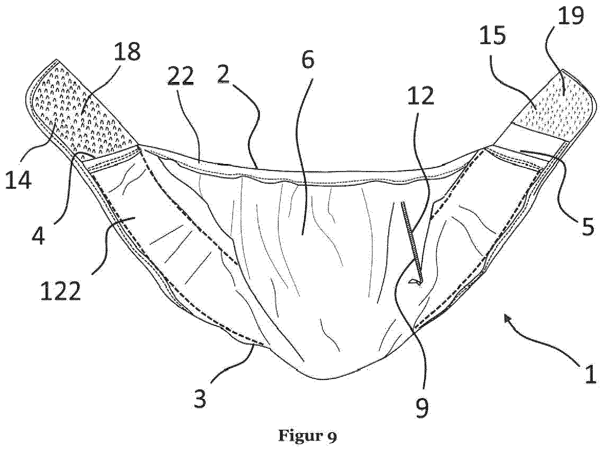

Figur 9 zeigt eine zu Figur 8 ähnliche Strahlenschutzbekleidung 1, wobei am unteren Ende ein breites Gummiband 122 eingearbeitet ist, welches den Bauch abstützt. Die Konturen des Gummibands 122 sind durch eine in Figur 8 nicht vorhandene gestrichelte Linie angedeutet. Das Gummiband 122 kann beispielsweise mindestens 3 cm breit sein. Besagtes Gummiband 122 ist vorzugsweise in Stoff eingeschlossen und liegt insbesondere zwischen der Außenlage 6 und der Innenlage vor. Vorzugsweise ist eine Abschirmlage über Abschirmlagefixierungsmittel, zweckmäßigerweise entlang von oberem und unterem Ende und gegebenenfalls auch entlang von erstem und zweitem Seitenrand oder entlang von erstem und zweitem Seitenrand und gegebenenfalls auch entlang von oberem und unteren Ende oder entlang von oberem und unterem Ende und auch entlang von erstem und zweitem Seitenrand, fixiert, wobei es sich um Haken, Ösen, Knöpfe und/oder Schnürungen handeln kann, insbesondere Druckknöpfe oder Knöpfe (nicht gezeigt). Die Abschirmlage ist vorzugsweise am unteren und am oberen Ende im Innenraum fixiert, insbesondere umlaufend fixiert (nicht gezeigt). Vorzugsweise erstreckt sich die Abschirmlage auch vom ersten bis zum zweiten Seitenrand (nicht gezeigt).

Die in der voranstehenden Beschreibung, den Ansprüchen sowie den Zeichnungen offenbarten Merkmale der Erfindung können sowohl einzeln als auch in jeder beliebigen Kombination für die Verwirklichung der Erfindung in ihren verschiedenen Ausführungsformen wesentlich sein. Die Figuren 1 bis 9 können sich auf verschiedene Ausführungsformen beziehen. Auch ist eine Ausführungsform denkbar, welche mehrerer oder aller der Figuren 1 bis 9 entspricht.

### Liste der Bezugszeichen

- 1: Strahlenschutzbekleidung
- 2: oberes Ende
- 3: unteres Ende
- 4: erster Seitenrand
- 5: zweiter Seitenrand
- 6: Außenlage
- 7: Innenlage
- 8: Innenraum
- 9: Eingriffsöffnung
- 10: Abschirmlage
- 11: Strahlenschutztextil
- 12: Verschlussmittel
- 13: Stoffschutzstreifen
- 14: erste Befestigungsmittel
- 15: zweite Befestigungsmittel
- 16: dritte Befestigungsmittel
- 17: vierte Befestigungsmittel
- 18: erstes Befestigungsband
- 19: zweites Befestigungsband
- 20: drittes Befestigungsband
- 21: viertes Befestigungsband
- 22: Silikon-Gummiband
- 23: erste Abschirmlagefixierungsmittel
- 24: zweite Abschirmlagefixierungsmittel
- 25: Bereich mit Innenraumnähten
- 26: erster elastischer Gewebestreifen
- 27: zweiter elastischer Gewebestreifen
- 28: Innenraumnähte
- 29: Raffung bzw. Falten des Strahlenschutztextils

## Patentansprüche

1. Strahlenschutzbekleidung (1), insbesondere Bauchbinde, vorzugsweise für den Schutz vor Mobilfunkstrahlung,
vorzugsweise umfassend ein oberes Ende (2), ein unteres Ende (3), einen ersten Seitenrand (4) und einen gegenüberliegenden zweiten Seitenrand (5),
ferner umfassend eine Außenlage (6) und eine Innenlage (7),
wobei die Strahlenschutzbekleidung (1) zwischen der Außenlage (6) und der Innenlage (7) einen Innenraum (8) mit einer Eingriffsöffnung (9) und eine Abschirmlage (10), insbesondere mit einem Strahlenschutztextil (11), aufweist, wobei die Abschirmlage (10) entfernbar in dem Innenraum (8) angeordnet ist.

2. Strahlenschutzbekleidung (1) nach Anspruch 1, **dadurch gekennzeichnet, dass** die Eingriffsöffnung (9) mit einem Verschlussmittel (12) verschließbar ist, insbesondere wobei das Verschlussmittel (12) ein Reißverschluss, ein Klettverschluss, ein Schnürverschluss, ein magnetischer Verschluss, ein Hakenverschluss oder ein Knopfverschluss ist.

3. Strahlenschutzbekleidung (1) nach Anspruch 1 oder 2, **dadurch gekennzeichnet, dass**
die Strahlenschutzbekleidung (1) am ersten Seitenrand (4) mindestens ein erstes Befestigungsmittel (14) aufweist, welches ausgelegt und eingerichtet ist, mit mindestens einem zweiten Befestigungsmittel (15) des zweiten Seitenrandes (5) verbunden zu werden.

4. Strahlenschutzbekleidung (1) nach einem der vorangehenden Ansprüche, insbesondere nach Anspruch 3, **dadurch gekennzeichnet, dass**
die Strahlenschutzbekleidung (1) an dem ersten Seitenrand (4) mindestens ein erstes Befestigungsband (18) mit einer Vielzahl von ersten Befestigungsmitteln (14) aufweist, insbesondere mindestens drei ersten Befestigungsmitteln, vorzugsweise mindestens zehn ersten Befestigungsmitteln, und/oder dass die Strahlenschutzbekleidung (1) an dem zweiten Seitenrand (5) mindestens ein zweites Befestigungsband (19) mit einer Vielzahl von zweiten Befestigungsmitteln (15) aufweist, insbesondere mindestens drei zweiten Befestigungsmitteln, vorzugsweise mindestens zehn zweiten Befestigungsmitteln.

5. Strahlenschutzbekleidung (1) nach Anspruch 3 oder 4, **dadurch gekennzeichnet, dass**
die Befestigungsmittel, insbesondere die ersten und/oder zweiten Befestigungsmittel (14, 15), Haken, insbesondere Metallhaken oder Klettverschlusshaken, Ösen, Knöpfe, insbesondere Druckknöpfe, und/oder Schnürungen umfassen oder sind.

6. Strahlenschutzbekleidung (1) nach einem der vorangehenden Ansprüche, **dadurch gekennzeichnet, dass**
die Strahlenschutzbekleidung (1) am oberen und/oder unteren Ende (2, 3) und/oder beabstandet von diesem, insbesondere am oberen Ende (2), ein Gummiband (22) aufweist, insbesondere bevorzugt wobei das Gummiband (22) sich vom ersten Seitenrand (4) oder aus Richtung des ersten Seitenrands (4) bis zum zweiten Seitenrand (5) oder in Richtung des zweiten Seitenrands (5) erstreckt.

7. Strahlenschutzbekleidung (1) nach einem der vorangehenden Ansprüche, **dadurch gekennzeichnet, dass**
die Abschirmlage (10), vorzugsweise randständig, mindestens ein erstes Abschirmlagefixierungsmittel (23) aufweist, welches ausgelegt und eingerichtet ist, mit mindestens einem zweiten Abschirmlagefixierungsmittel (24), das der Innenraum (8) aufweist, verbunden zu werden.

8. Strahlenschutzbekleidung (1) nach Anspruch 7, **dadurch gekennzeichnet, dass** der Innenraum (8) einen, insbesondere randständigen, Bereich mit Innenraumnähten (28) umfasst, wobei die zweiten Abschirmlagefixierungsmittel (24) mit besagtem Bereich verbunden sind, insbesondere mit den Innenraumnähten (28) in Kontakt stehen, vorzugsweise wobei besagte Innenraumnähte (28) die Innenlage (7) und die Außenlage (6) miteinander verbindet.

9. Strahlenschutzbekleidung (1) nach Anspruch 7 oder 8, **dadurch gekennzeichnet, dass**
die Abschirmlage (10), insbesondere randständig, einen ersten elastischen Gewebestreifen (26) umfasst, wobei das mindestens eine erste Abschirmlagefixierungsmittel (23) an dem ersten elastischen Gewebestreifen (26) befestigt ist und/oder dass der Innenraum (8) einen zweiten elastischen Gewebestreifen (27) umfasst, wobei das mindestens eine zweite Abschirmlagefixierungsmittel (24) an dem zweiten elastischen Gewebestreifen (27) befestigt ist.

10. Strahlenschutzbekleidung (1) nach einem der Ansprüche 7 bis 9, **dadurch gekennzeichnet, dass**
die ersten und zweiten Abschirmlagefixierungsmittel (23, 24) jeweils gegenüberliegende Bestandteile von Knöpfen, insbesondere Druckknöpfen sind.

11. Strahlenschutzbekleidung (1) nach einem der vorangehenden Ansprüche, **dadurch gekennzeichnet, dass**
die Abschirmlage (10), insbesondere randständig, einen ersten elastischen Gewebestreifen (26), vorzugsweise den ersten elastischen Gewebestreifen (26) nach Anspruch 21, umfasst, wobei der erste elastische Gewebestreifen (26) das Strahlenschutztextil (11) der Abschirmlage (10) umschließt, wobei das Strahlenschutztextil (11) in einem relaxierten und/oder entspannten Zustand des ersten elastischen Gewebestreifens (26) gerafft ist und/oder mehrere Falten (29) aufweist und erst durch Dehnung des ersten elastischen Gewebestreifens (26) in einem Zustand ohne besagte Raffung bzw. mit weniger Falten (29) überführbar ist, insbesondere wobei das Strahlenschutztextil (11) weniger stark dehnbar als der erste elastischen Gewebestreifen (26) ist.

12. Strahlenschutzbekleidung (1) einem der vorangehenden Ansprüche, **dadurch gekennzeichnet, dass**
die Innenlage (7) und die Außenlage (6) am oberen Ende (2), am unteren Ende (3), am ersten Seitenrand (4) und/oder am zweiten Seitenrand (5) durch, insbesondere elastische, Innenraumnähte (28) miteinander verbunden sind, insbesondere wobei die Innenraumnähte (28) auch den zweiten elastischen Gewebestreifen (27) fixieren.

13. Strahlenschutzbekleidung (1) nach einem der vorangehenden Ansprüche, **dadurch gekennzeichnet, dass**
das Strahlenschutztextil (11) ein Metalldrahtgeflecht umfasst, insbesondere ein ein Silbergewebe umfasst oder darstellt und/oder antistatisch, antiseptisch, faltbar und/oder knickbar ist.

14. Strahlenschutzbekleidung (1) nach einem der vorangehenden Ansprüche, **dadurch gekennzeichnet, dass**
das Strahlenschutztextil bei 900 MHz, 1800 MHz und/oder bei 2600 MHz einen Schirmdämpfungswert von mindestens 10 dB, insbesondere mindestens 40 dB, vorzugsweise mindestens 50 dB, aufweist.

15. Strahlenschutzbekleidung (1) nach einem der Ansprüche 2 bis 14, **dadurch gekennzeichnet, dass**
das Verschlussmittel (12) in der Außenlage (6) vorliegt.
